# FASCICULE DE BREVET EUROPEEN

(11) **EP 4 135 793 B1**
(45) Date de publication et mention de la délivrance du brevet: **21.10.2026**
(21) Numéro de dépôt: 21726694.9
(22) Date de dépôt: 15.04.2021
(51) Int. Cl.: A61M 5/19, A61M 5/31, A61M 39/24, A61M 5/20, A61M 39/22, A61M 5/34

(54) **SERINGUE D'INJECTION**
INJEKTIONSSPRITZE
INJECTION SYRINGE

(30) Priorité: 15.04.2020 FR 2003755
(43) Date de publication de la demande: 22.02.2023
(73) Titulaire: AH2I Medical Group, Ramat-Gan (IL)
(72) Inventeur: KOROLIK, Pavel, 4975205 Petah Tiqva (IL); LIBERZON, Alexander, 4265491 Netanya (IL); KOGAN, Inna, 4975205 Petah Tiqva (IL)
(74) Mandataire: Oak & Fox
(86) Numéro de dépôt international: PCT/FR2021/050664
(87) Numéro de publication internationale: WO 2021/209721

(56) Documents cités:
- WO-A1-2012/139035
- WO-A1-2017/120358
- WO-A1-2019/243853
- US-A1- 2011 118 659
- US-A1- 2015 112 248
- US-A1- 2017 304 553

## Description

### Domaine technique.

L'invention a pour objet une seringue permettant d'injecter de manière sécurisée, un produit dans un site d'injection corporel d'un individu.

L'invention se rapporte au domaine technique des systèmes et appareils d'injection utilisés notamment dans le domaine médical, en dermatologie ou en cosmétologie.

### Etat de la technique.

Il est courant, dans le domaine de la dermatologie ou de la cosmétique, d'utiliser des produits de comblement dermique. Ce produit est administré par injection au moyen d'une seringue, généralement dans le plan supra-périosté, dans ou au-dessus du coussinet adipeux sous-cutané. Ces injections font partie des procédures peu invasives les plus courantes pratiquées par les dermatologues et les chirurgiens plasticiens afin de palier à l'affaissement de la peau et/ou de combler les rides.

L'administration du produit de comblement est parfois compliquée par des effets négatifs tels que : un gonflement, une nécrose ou une anaphylaxie. Il existe également d'importantes complications vitales rapportées dans la littérature, comme une déficience visuelle, une nécrose de la peau ou une cécité.

Le danger le plus important provient de l'injection intra-vasculaire. Les règles générales pour des injections sûres comprennent l'aspiration de sang avant l'injection pour des raisons de sécurité. Plus particulièrement, on élimine préalablement l'air de la seringue en amorçant (ou purgeant) l'aiguille avec le produit à injecter. Lorsque l'aiguille est insérée dans la peau de l'individu, le praticien effectue un mouvement de retrait du piston pour aspirer un échantillon de fluide corporel (ci-après définie comme l'aspiration in-vivo). Le praticien observe alors visuellement si l'échantillon aspiré contient du sang. Si tel est le cas, le produit sera injecté dans un vaisseau sanguin, avec les risques décrits précédemment. Il convient dans ce cas de modifier le site d'injection.

L'aspiration de sang avec les aiguilles utilisées pour l'injection d'un produit de comblement (généralement des aiguilles de gauge 29G ou 31G) n'est pas pratique en raison de la viscosité dynamique élevée des produits de comblement utilisés. Aussi, il arrive fréquemment que le praticien ne puisse tout simplement pas réaliser l'aspiration préalable de l'échantillon de fluide corporel et ne puisse donc pas vérifier si l'aiguille atteint ou pas un vaisseau sanguin.

En outre, la purge de l'air contenu dans l'aiguille, par le produit actif à injecter, entraine une perte partielle dudit produit. Or ces produits sont généralement onéreux.

De la demande de brevet US2017304553 et de la demande de brevet et de la demande de brevet WO2017120358, on connaît des solutions comprenant indicateur de test sensible à un fluide particulier. Des demandes de brevet WO2012/139035 A1, WO 2019/2433853 A1, US2011/118659 A1, US 2015/112248 A1, on connaît des assemblages de deux seringues destinées à l'injection et/ou à l'aspiration d'un fluide.

L'invention vise à remédier à cet état des choses. Plus particulièrement, l'invention vise à proposer une seringue d'injection permettant d'atteindre au moins l'un des objectifs suivants :
- permettre des injections sures et fiables,
- assurer de manière simple et efficace l'aspiration in-vivo avant l'injection du produit actif,
- réduire les pertes de produit actif à injecter,
- proposer une seringue de conception simple, peu onéreuse à fabriquer, fiable et facile d'utilisation.

### Présentation de l'invention.

La solution proposée par l'invention est définie par la revendication indépendante. Les revendications dépendantes définissent des modes de réalisation de l'invention.

Ainsi, la seringue d'injection selon la présente invention offre la possibilité avantageuse de vérifier avant l'injection du premier produit injectable que l'aiguille creuse ne se trouve pas introduite dans un vaisseau sanguin ((par ex. artère, veine, capillaire) présent dans le site d'injection. Cela permet d'éviter les risques d'injection intra-vasculaire accidentelle de certains produits injectables tels que les produits de comblement dermique, les anesthésiants locaux ou autres produits dont l'injection intra-vasculaire est à éviter.

Aussi, l'utilisation d'une seringue à deux corps de seringue permet de réaliser l'aspiration *in-vivo* (en manipulant le second corps de seringue) pour s'assurer que l'injection du produit actif ne sera pas une injection intra-vasculaire, tout en permettant, avec le même appareil, et au travers de la même aiguille, d'injecter en toute sécurité le produit actif (en manipulant le premier corps de seringue).

### Brève description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
[Fig. 1] est une vue éclatée schématique d'une seringue conforme à l'invention, selon un premier mode de réalisation,
[Fig. 2] est une vue en perspective d'une seringue conforme à l'invention selon un deuxième mode de réalisation,
[Fig. 3] est une vue éclatée de la seringue de la figure 2,
[Fig. 4a] est une vue en coupe d'un adaptateur selon un premier mode de réalisation, l'obturateur étant en position fermée,
[Fig. 4b] montre l'adaptateur de la figure 4a, l'obturateur étant en position ouverte,
[Fig. 5a] schématise la répartition des deux produits de la seringue dans l'adaptateur des figures 4a et 4b, dans une configuration,
[Fig. 5b] schématise la répartition des deux produits de la seringue dans l'adaptateur des figures 4a et 4b, dans une autre configuration,
[Fig. 5c] schématise la répartition des deux produits de la seringue dans l'adaptateur des figures 4a et 4b, dans une autre configuration,
[Fig. 5d] schématise la répartition des deux produits de la seringue dans l'adaptateur des figures 4a et 4b, dans une autre configuration,
[Fig. 6a] schématise la répartition des deux produits de la seringue dans un adaptateur selon un second mode de réalisation, dans une configuration,
[Fig. 6b] schématise la répartition des deux produits de la seringue dans l'adaptateur de la figure 6a, dans une autre configuration,
[Fig. 7a] schématise la répartition des deux produits de la seringue dans un adaptateur selon un troisième mode de réalisation, dans une configuration,
[Fig. 7b] schématise la répartition des deux produits de la seringue dans l'adaptateur de la figure 7a, dans une autre configuration,
[Fig. 8a] schématise la répartition des deux produits de la seringue dans un adaptateur selon un quatrième mode de réalisation, dans une configuration,
[Fig. 8b] schématise la répartition des deux produits de la seringue dans l'adaptateur de la figure 8a, dans une autre configuration,
[Fig. 9a] schématise la répartition des deux produits de la seringue dans un adaptateur selon un cinquième mode de réalisation, dans une configuration,
[Fig. 9b] schématise la répartition des deux produits de la seringue dans l'adaptateur de la figure 9a, dans une autre configuration,
[Fig. 10a] montre une seringue conforme à l'invention, selon un troisième mode de réalisation, et selon une première configuration d'utilisation,
[Fig. 10b] montre la seringue de la figure 10a, selon une seconde configuration d'utilisation.
[Fig. 11a] est une vue en coupe en perspective d'un adaptateur selon un sixième mode de réalisation, l'obturateur étant logé dans une cavité aménagée à l'intersection entre les trois voies de communication fluide de l'adaptateur,
[Fig. 11b] schématise la répartition des deux produits de la seringue dans l'adaptateur de la figure 11a, dans une configuration,
[Fig. 11c] schématise la répartition des deux produits de la seringue dans l'adaptateur de la figure 11a, dans une autre configuration,

### Description des modes de réalisation.

Par souci de clarté, tel qu'utilisé ici, sauf indication contraire, l'utilisation des adjectifs ordinaux « premier », « deuxième », etc., pour décrire un objet indique simplement que différentes occurrences d'objets similaires sont mentionnées et n'implique pas que les objets ainsi décrits doivent être dans une séquence donnée, que ce soit dans le temps, dans l'espace, dans un classement ou de toute autre manière. De même, l'utilisation des adjectifs « proximal(e)/distal(e) », « supérieur(e)/inférieur(e) », droite/gauche », « avant/arrière » etc., permet de décrire simplement la position d'un objet dans la configuration des figures annexées, mais n'implique pas, qu'en pratique, des objets similaires soient dans la même position.

La seringue objet de l'invention est particulièrement adaptée pour la mise en œuvre d'un traitement non thérapeutique, dermatologique ou cosmétologique, comprenant une étape préalable d'aspiration in-vivo et une étape d'injection d'un produit actif dans un site d'injection corporel d'un individu, tel qu'un produit de comblement dermique. La mise en œuvre d'un tel traitement est particulièrement efficace lorsque l'aspiration in-vivo est réalisée avec un fluide moins visqueux que le produit actif injecté dans le site d'injection.

La seringue peut toutefois être utilisée avec des produits ayant des viscosités dynamiques proches et/ou dans le cadre d'un traitement thérapeutique, notamment pour l'injection sûre d'une formulation médicale injectable, par exemple dans le cas de chirurgies vasculaires, de thérapies veineuses, de traitement local de la douleur, d'injections par voies intrathécales ou épidurales, etc.

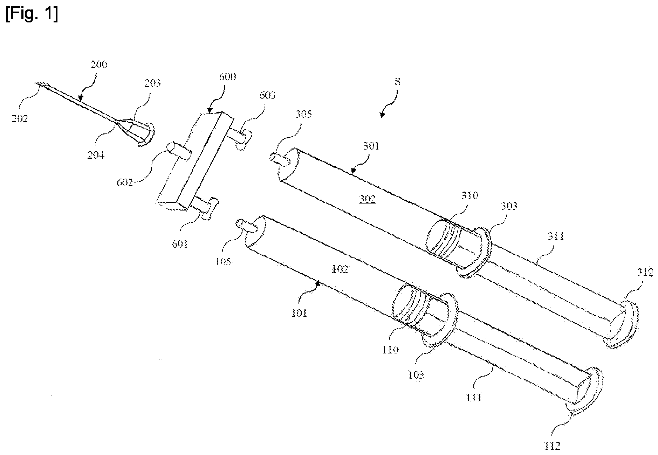

Sur la figure 1, la seringue S comprend deux corps de seringue, respectivement 101 et 301. Chaque corps de seringue est préférentiellement de forme cylindrique, dont la paroi interne définie une chambre 102, 302 dont la contenance est par exemple comprise entre 1 mL et 20 mL. Les corps de seringue 101 et 301 peuvent être de différents types et/ou de différentes tailles. Chaque corps de seringue 101, 301 présente une extrémité proximale 105, 305 (ou embout de décharge) par laquelle le produit s'écoule hors de la chambre102, 302, et une extrémité distale 103, 303 opposée à ladite extrémité proximale. Les corps de seringue 101, 301 peuvent être réalisé dans un matériau plastique, en acier, en verre, ou dan tout autre matériau convenant à l'homme du métier.

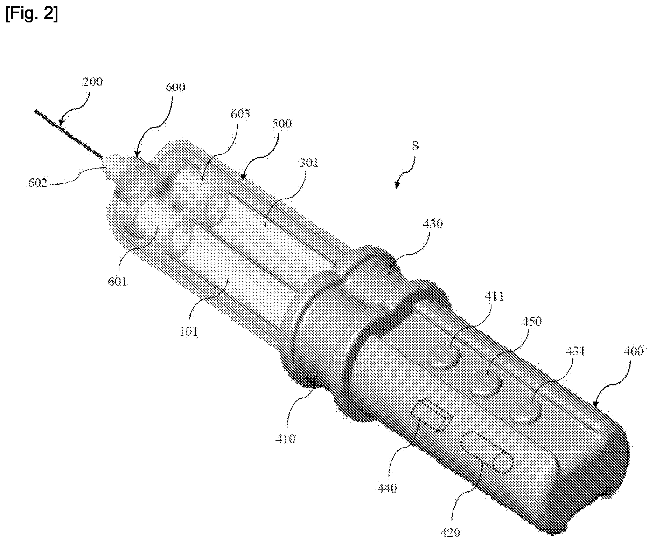

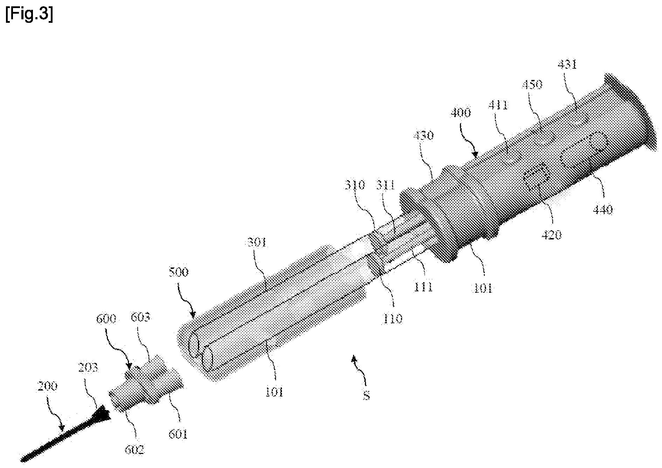

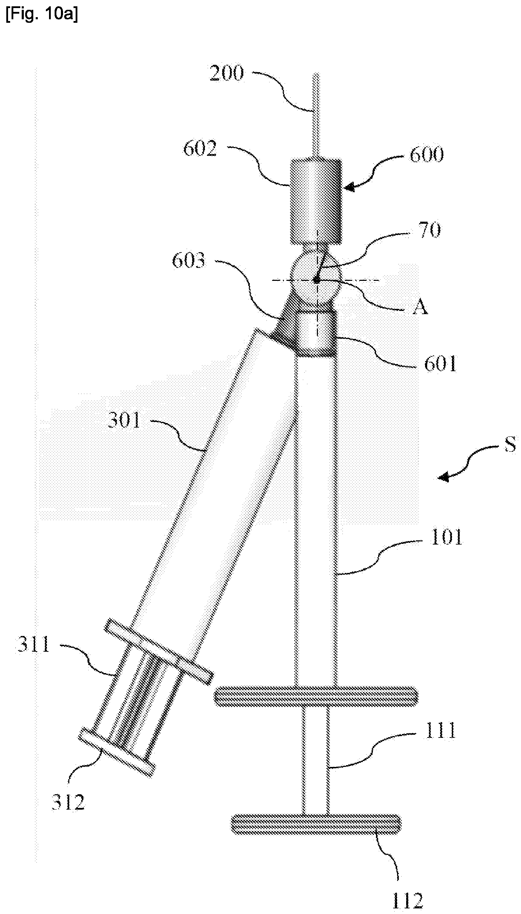

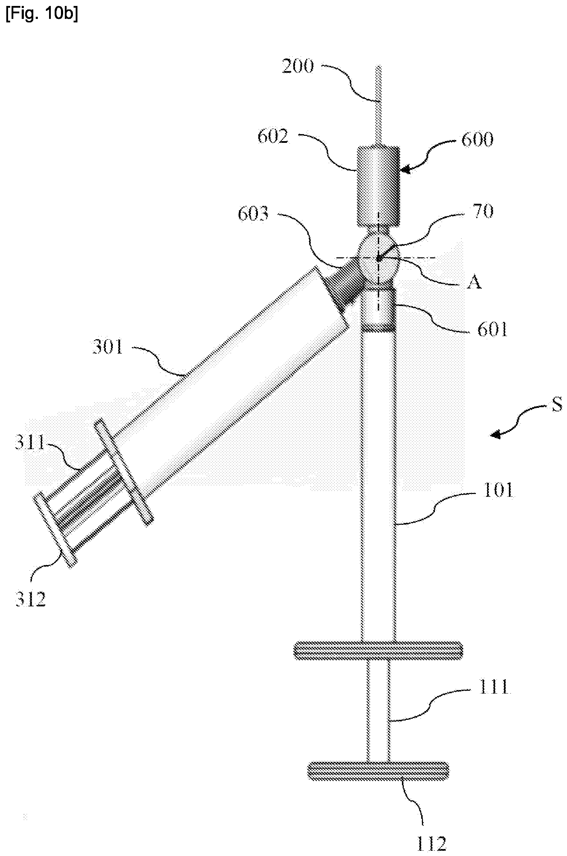

Les deux corps de seringue 101, 301 peuvent être laissés libres (figures 1, 10a, 10b) ou être installé dans un capot 500 qui les protège (figures 2 et 3). Au moins la partie du capot 500 en regard des corps de seringue 101, 301, est préférentiellement réalisé dans un matériau transparent, par exemple en verre ou en plastique, de sorte que l'utilisateur peut contrôler le volume restant des produits F1 et F2.

Le premier corps de seringue 101 (plus particulièrement sa chambre 102) est destiné à contenir un premier produit injectable F1 et le second corps de seringue 301 (plus particulièrement sa chambre 302) est destiné à contenir un second produit F2. Avant utilisation de la seringue S, les deux corps de seringues 101, 301 sont pré-remplis avec leur produit respectifs, F1, F2, selon toutes méthode appropriée et conne de l'homme du métier.

Pour une application dermatologique ou cosmétique, le premier produit F1 est préférentiellement un produit de comblement dermique se présentant sous la forme d'une solution injectable (liquide, gel, particules en suspension). Ce produit F1 comprend au moins une substance sélectionnée dans le groupe constitué de: acide hyaluronique, collagène, acide poly-lactique, gel de polyacrylamide, hydroxyapatite de calcium, microsphères de calcium hydroxyl appétit en suspension dans un support de gel aqueux, phosphate tricalcique, microsphères de polyméthacrylate de méthyle, billes de dextran, billes de dextran suspendues dans du gel hylan d'origine non animale, peptides d'élastine solubilisés avec du collagène bovin, silicone, acide poly-L-lactique, polytétrafluoroéthylène (PTFE), polytétrafluoroéthylène expansé (e-PTFE) et au moins l'une de leurs combinaisons. Il peut également comprendre un ou plusieurs agents biologiquement actifs tels que les toxines botuliques, les anesthésiants locaux, les corticoïdes locaux, ou les antibiotiques, les acides aminés, les antioxydants.

Pour une application thérapeutique, le premier produit F1 peut être une formulation médicale se présentant sous la forme d'une solution liquide injectable (liquide, particules en suspension). Cette formulation médicale injectable comprend notamment un ou plusieurs agents biologiquement actifs sélectionnés dans le groupe constitué de : toxines botuliques, anesthésiants locaux, corticoïdes locaux, antibiotiques, acides aminés, antioxydants, produits opacifiants, et au moins l'une de leurs combinaisons.

Le second produit F2 est préférentiellement un produit injectable neutre (c'est-à-dire sans effet notoire sur l'individu d'un point de vue dermatologique, cosmétique, ou thérapeutique), notamment une solution liquide saline physiologique, par exemple une solution composée d'eau distillée et de chlorure de sodium (NaCl) dilué de 1/1000 à 10/1000.

Selon un mode préféré de réalisation, les deux produits F1 et F2 n'ont pas la même viscosité dynamique. En particulier, le second produit F2 est moins visqueux que le premier produit actif F1. Par exemple, à température et pression ambiantes, le premier produit F1 peut avoir une viscosité dynamique comprise entre 0,1 Pa.s et 1000 Pa.s (N.m⁻².s) et le second produit F2 une viscosité dynamique comprise entre 10⁻³ Pa.s et 10⁻² Pa.s. L'utilisation d'un second produit F2 peu visqueux, permet de réaliser l'étape d'aspiration in-vivo de manière très simple, sans résistance particulière.

Dans la suite de la description, et par souci de clarté et de concision seulement, on appellera le premier produit F1 : le fluide de comblement et le second produit F2 : la solution saline. La solution saline a une viscosité dynamique plus faible que le fluide de comblement.

De manière classique, un premier piston 110 est monté déplaçable dans le premier corps 101 et un second piston est monté déplaçable dans le second corps 301. Chaque piston 110, 310 s'insère depuis l'extrémité distale 103, 303 du corps de seringue 101, 301 respectif et est monté mobile en translation dans ledit corps. Lorsqu'un piston 110, 310 est poussé vers l'avant, c'est-à-dire qu'il est déplacé vers l'extrémité proximale 105, 305 du corps de seringue 101, 301, le volume de la chambre 102, 302 diminue et la pression à l'intérieur dudit corps augmente. Le produit F1, F2 peut alors être évacué hors du corps de seringue, par son extrémité proximale 105, 305. A l'inverse, lorsqu'un piston 110, 310 est tiré vers l'arrière - ou rétracté, c'est-à-dire qu'il est déplacé vers l'extrémité distale 103, 303 du corps de seringue 101, 301, le volume de la chambre 102, 302 augmente et la pression à l'intérieur dudit corps diminue. Cette mise en dépression permet d'aspirer un fluide depuis l'extrémité proximale 105, 305.

Les pistons 110, 310, sont réalisés en plastique ou en métal. Ils sont avantageusement pourvus de joints d'étanchéité sur leur paroi externe. Ils sont adaptés à leur corps de seringue respectif et peuvent être de différents types et/ou de différentes tailles.

Le second piston 310 est manœuvrable indépendamment du premier piston 110. En d'autres termes, les deux pistons peuvent être manipulés indépendamment l'un de l'autre. On peut donc injecter de manière indépendante chaque produit F1 ou F2 et/ou faire varier de manière indépendante le volume des chambres 102, 302, pour augmenter ou diminuer de manière indépendante la pression dans chaque corps 101, 301.

Dans le mode de réalisation des figures 1, 10a et 10b, les pistons 110, 310 sont manœuvrables manuellement. Ils sont chacun relié à une tige 111, 311 (en plastique ou en acier) s'étendant chacune à partir d'un poussoir 112, 312. En tirant ou en poussant manuellement le poussoir 112, 312 avec ses doigts, le praticien peut déplacer le piston correspondant 110, 310 à l'intérieur du corps de seringue 101, 301.

Dans le mode de réalisation des figures 2 et 3, les pistons 110, 310 sont mus par des moyens d'entraînement motorisés 410, 430, par exemple de type à vis sans fin ou crémaillère mis en rotation par un ou plusieurs moteurs rotatifs électriques éventuellement associés à un ou plusieurs pignons d'entrainement. Les moyens d'entraînement 410, 430 sont installés dans un châssis de protection 400 qui intègre également une batterie électrique 420 (de type pille ou batterie rechargeable) et une unité de gestion électronique 440. Des boutons de commande 411, 431, ou d'autres touches dédiées, sont installés sur la paroi externe du châssis 400 de manière à pouvoir être sélectionnés par l'utilisateur pour contrôler le déplacement des pistons 110, 310.

Comparé au mode de réalisation des figures 1, 10a et 10b, ce mode d'actionnement motorisé permet de déplacer de manière plus précise chacun des pistons 110, 310 et donc de dispenser de manière optimale le produit F1 et/ou le produit F2. Les pistons 110, 310 peuvent être entrainés de manière continue ou discontinue selon el besoin.

Le châssis 400 est avantageusement solidarisé au capot 500 de manière à ce que l'ensemble forme un appareil monobloc, particulièrement ergonomique et facilement manipulable par l'utilisateur. Le châssis 400 peut en outre comprendre un bouton de mise en tension de la seringue (bouton marche/arrêt) et, le cas échéant, des moyens d'affichage appropriés donnant le niveau de charge de la batterie électrique 420 et/ou le niveau de remplissage des corps de seringues 101, 301.

Selon un mode de réalisation, l'unité de gestion électronique 440 est programmée pour déplacer automatiquement les pistons 110, 310, de manière synchrone ou séquentielle, selon des cycles prédéfinis. Par exemple, l'exécution séquentielle des phases de purge et/ou d'aspiration in-vivo décrites plus avant dans la description peut être préprogrammée. Il en est de même de l'activation séquencée du ou des obturateurs décrits plus avant dans la description.

La seringue S comprend également une aiguille creuse 200 qui est unique et commune à chacun des corps de seringue 101, 301. L'aiguille 200 est préférentiellement une aiguille hypodermique classique, mais peut se présenter sous la forme d'une une micro-canule. A titre d'exemple, l'aiguille 200 a un calibre - ou gauge - allant de 18G à 34G, plus particulièrement, de 25G à 30G. Sa longueur est par exemple comprise entre 0,5 cm et 4 cm. L'extrémité distale 202 de l'aiguille 200 est de forme biseautée, telle que connue de l'homme du métier, afin de faciliter son insertion dans le site d'injection. Un porte-aiguille 203 est avantageusement monté sur l'extrémité proximale de l'aiguille 200. L'utilisateur peut ainsi aisément changer d'aiguille.

Un adaptateur 600 est connecté au premier corps de seringue 101, au second corps de seringue 301 et à l'aguille 200. Cet adaptateur 600 peut être réalisé en matériau plastique, notamment des plastiques compatibles pour les applications non-thérapeutique et thérapeutique de l'invention tels que Acrylonitrile Butadiène Styrène (ABS) ; nylon ; Polycarbonate (PC) ; Polypropylène (PP) ; poly(chlorure de vinyle) (PVC). L'adaptateur 600 peut encore être réalisé en inox, en acier, etc. Il comprend un premier port d'entrée 601 en communication fluidique avec le premier corps de seringue 101, un second port d'entrée 603 en communication fluidique avec le second corps de seringue 301, et un port de sortie 602 en communication fluidique avec l'aguille 200. A titre d'exemple, l'adaptateur 60 peut être contenu dans une enveloppe de dimensions 30mm x 35mm x 10mm.

Sur la figure 1, le premier port d'entrée 601 est connecté de manière démontable sur l'extrémité proximale 105 du premier corps de seringue 101. La connexion peut comprendre un dispositif de verrouillage du type Luer-Lock. La connexion du second port d'entrée 601 sur l'extrémité proximale 305 du second corps de seringue 301 est similaire. Sur les figures 2 et 3, les ports d'entrée 601, 603 sont emboîtés de manière étanche aux extrémités proximale 105, 305. Sur les figures 1 à 3, le port de sortie 602 est connecté de manière démontable au porte-aiguille 203, par exemple au moyen d'un dispositif de verrouillage du type Luer-Lock.

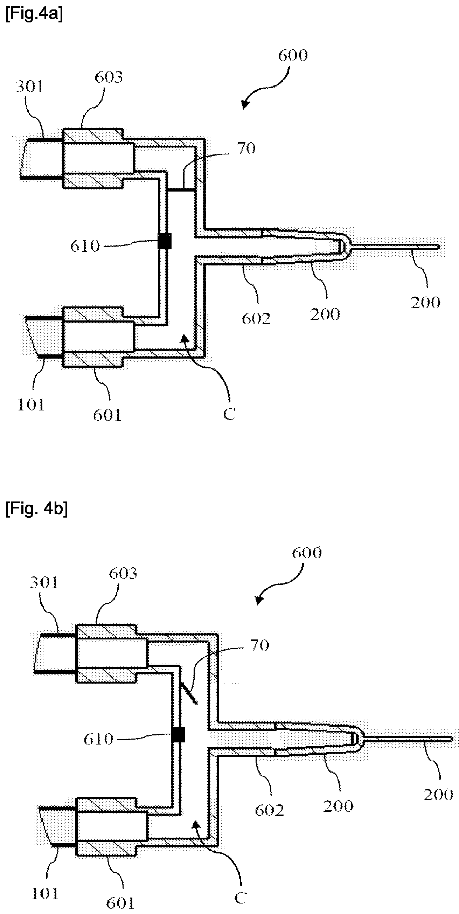

Sur la figure 4a, l'adaptateur 600 présente un conduit interne C adapté pour mettre en communication fluidique le premier port d'entrée 601, le deuxième port d'entrée 603 et le port de sortie 605. A titre d'exemple, le conduit C a une section circulaire dont le diamètre interne est compris entre 0,1 mm et 10 mm. En pratique, la section et/ou le diamètre interne du conduit C sont choisis en fonction du type de produit à injecter et/ou de sa dose d'injection. Sur les figures annexées, le conduit C à la forme d'un T ou d'un Y, dont la branche principale débouche au niveau du port de sortie 602 et dont chaque branche latérale débouche respectivement au niveau d'un port d'entrée 601, 603.

L'adaptateur 600 comprend au moins un obturateur coopérant avec le conduit C. Cet obturateur est configuré pour alternativement autoriser ou empêcher : la communication fluidique entre le premier port d'entrée 601 et le port de sortie 602, et/ou la communication fluidique entre le second port d'entrée 603 et le port de sortie 602. En actionnant spécifiquement cet obturateur, l'utilisateur peut aisément fermer de manière étanche une portion du conduit C pour isoler l'un ou l'autre des corps de seringue 101, 301 et contrôler le produit à faire passer au travers de l'aiguille 200.

Différents modes de réalisation de cet obturateur vont maintenant être décrits en références aux différentes figures annexées. Différents séquences d'utilisation de la seringue S sont également décrits en référence à ces figures.

### Premier mode de réalisation : figures 4a à 5d

L'adaptateur 600 comporte un seul obturateur 70 mobile entre une position ouverte (figure 4a) autorisant la communication fluidique entre le second port d'entrée 603 et le port de sortie 602 et une position fermée (figure 4b) empêchant la communication fluidique entre ledit second port d'entrée et ledit port de sortie. Cet obturateur 70 est disposé au niveau du second port d'entrée 603, dans la branche du conduit C associée audit second port. L'autre branche du conduit C associée au premier port d'entrée 601 est libre, c'est-à-dire dépourvue d'obturateur ou d'autre organe assurant cette fonction.

Cet obturateur 70 se présente sous la forme d'un clapet coopérant avec un siège de clapet installé dans le conduit C. Lorsque le clapet est en appui contre son siège, le conduit C est fermé de manière étanche. Et lorsque le clapet est à distance de son siège, le produit peut s'écouler librement dans le conduit C. Ce clapet peut être un clapet anti-retour autorisant le passage du produit que dans un sens pour éviter le mélange des produits F1, F2. Selon un autre mode de réalisation le clapet 70 est intégré dans une vanne ou une électrovanne installée dans le conduit C.

Selon un mode de réalisation, le clapet 70 est activé manuellement, par exemple au moyen d'un bouton poussoir ou d'une molette, adapté pour le faire basculer entre les positions ouverte et fermée. Dans le mode de réalisation des figures 10a et 10b, le second corps de seringue 301 est monté pivotant sur l'adaptateur 600, autour d'un axe A perpendiculaire à l'axe dudit corps. Dans la position de la figure 10a, le clapet 70 est dans la position ouverte. En faisant pivoter le second corps de seringue 301 autour de l'axe A, pas exemple dans un sens horaire qui l'éloigne du premier corps de seringue 101, le clapet 70 est dans la position fermée (figure 10b). L'utilisateur doit donc basculer le second corps de seringue 301 autour de l'axe A pour ouvrir ou fermer le clapet 70.

Selon un autre mode de réalisation, le clapet 70 est intégré dans le dispositif de verrouillage du type Luer-Lock permettant de connecter l'extrémité proximale 305 du second corps de seringue 301 au second port d'entrée 603. En faisant pivoter ce dispositif dans un sens, par exemple de ¼ de tour ou ½ tour dans le sens horaire, le clapet 70 est placé en position ouverte. Et en faisant pivoter ce dispositif dans l'autre sens, par exemple de ¼ de tour ou ½ tour dans le sens anti-horaire, le clapet 70 est placé en position fermée.

Selon un autre mode de réalisation, le clapet 70 est déplacé au moyen d'un actionneur électromécanique qui, peut par exemple se présenter sous la forme d'un moteur électrique ou d'un électro-aimant, dont l'alimentation en courant est pilotée par l'unité de gestion électronique 440. Dans ce cas, la commutation du clapet 70 entre les positions ouverte et fermée peut être commandée par l'activation d'un bouton de commande dédié 450 installé sur le châssis 400 (figures 2 et 3). Le clapet 70 peut encore être manœuvré au moyen d'un actionneur hydraulique ou pneumatique se présentant par exemple sous la forme d'un vérin dont la tige est reliée audit clapet.

Selon un autre mode de réalisation, le clapet 70 est un élément d'une valve micro-fluidique adaptée pour se déplacer sous l'effet de la pression. Par exemple, lorsque le second piston 310 est poussé vers l'avant, la pression à l'intérieur du second corps 301 augmente, ce qui tend à faire circuler la solution saline F2 hors dudit corps, pour pénétrer dans le conduit C. La solution saline F2 exerce alors une pression de poussée sur le clapet 70 qui tend à l'ouvrir, de sorte que ladite solution saline peut circuler du second port d'entrée 603 vers le port de sortie 602. L'utilisation d'une valve micro-fluidique permet de rendre le clapet 70 passif, c'est-à-dire qu'il ne nécessite pas d'actionneur pour le manœuvrer.

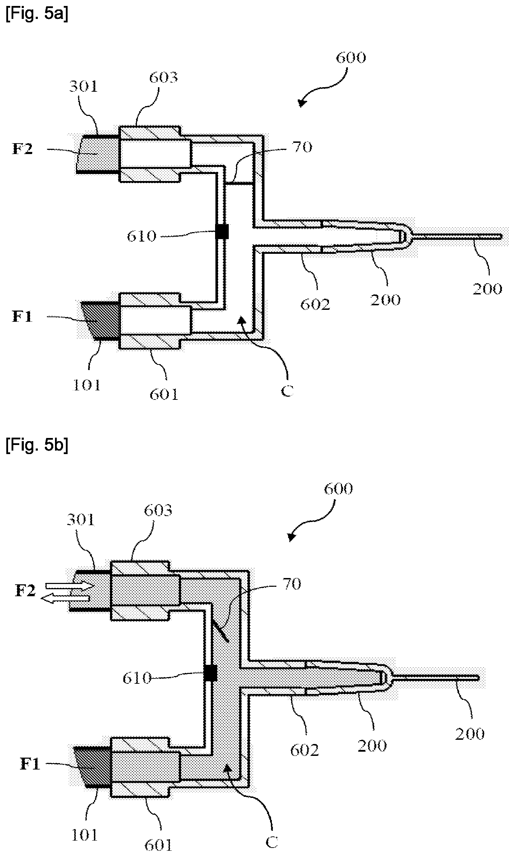

La séquence d'utilisation de la seringue S équipée de l'adaptateur 600 des figures 4a et 4b va maintenant être décrit en références aux figures 5a à 5d.

Figure 5a. La seringue S est dans un état initial : le clapet 70 est en position fermée et les deux pistons 110, 310 au repos.

Figure 5b. Pour purger l'air dans l'aiguille 200, le clapet 70 est placé en position ouverte, et le second piston 310 est poussé vers l'avant. La solution saline F2 s'écoule alors entre le second port d'entrée 603 et le port de sortie 602 de manière à purger l'air contenu dans l'aiguille. Cette purge préalable est donc réalisée avec un liquide neutre particulièrement bon marché, et en tout état de cause, n'est pas réalisée avec le fluide de comblement F1. La purge étant effectuée, l'aiguille 200 est alors insérée dans le site d'injection.

L'aspiration in-vivo peut être aisément réalisée dans la configuration de la figure 5b. Le second piston 310 est tiré vers l'arrière de manière à créer une dépression dans le conduit C, au niveau du second port d'entrée 603. La solution saline F2 contenue dans le conduit C et l'aiguille 200, reflue alors depuis le port de sortie 602 vers le second port d'entrée 603, tout en provoquant une aspiration d'un échantillon de fluide corporel. Ce fluide corporel est aspiré sans risque de se mélanger avec le fluide de comblement F1. En d'autres termes, le fluide de comblement F1 n'est pas souillé par le fluide corporel aspiré.
Toutefois, cette aspiration in-vivo peut également être réalisée, avec plus de force toutefois et avec un risque de souiller le fluide de comblement F1, en fermant le clapet 70 et en tirant vers l'arrière le premier piston 110 de manière à créer une dépression dans le conduit C, au niveau du premier port d'entrée 601.

Dans les deux cas, la solution saline F2 ayant une viscosité dynamique peu élevée, cette aspiration in-vivo est réalisée sans aucune difficulté, la résistance pour tirer vers l'arrière le piston étant très faible, même avec une aiguille de faible diamètre (ex : aiguille 18G à 34G). L'utilisateur peut alors vérifier que du sang n'a pas pénétré dans le corps de seringue par lequel est réalisée l'aspiration. Cette vérification peut être visuelle, l'utilisateur constate une coloration rougeâtre de la solution saline (respectivement du fluide de comblement), si du sang est aspiré dans le second corps de seringue 301 (respectivement dans le premier corps de seringue 101). Cette vérification peut également être automatique, notamment en intégrant un détecteur de sang 610 dans la seringue S. Ce détecteur 610 est préférentiellement connecté à l'unité de gestion électronique 440 (figures 3 et 4). Il est préférentiellement installé dans l'adaptateur 600 (figures 4a à 5d) et/ou dans le premier corps de seringue 101 et/ou dans le second corps de seringue 301. Ce détecteur 610 peut être basé sur une détection d'un composant du sang, par exemple sur une détection du glucose. Lorsque du glucose est détecté dans la seringue S (dans l'adaptateur 600 et/ou dans l'un des corps de seringue 101, 301), alors l'unité de gestion électronique 440 peut émettre un signal visuel et/ou sonore indiquant à l'utilisateur que l"aiguille 200 est insérée dans un vaisseau sanguin.

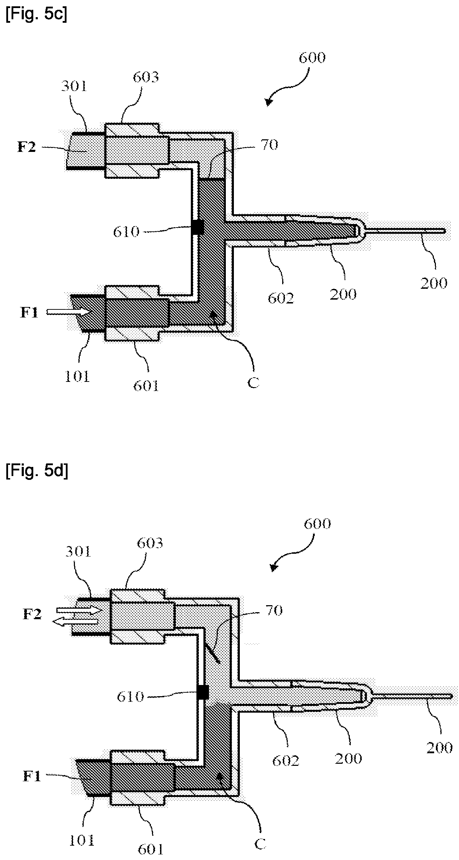

Figure 5c. L'utilisateur s'étant assuré que l'aguille 200 n'est pas insérée dans un vaisseau sanguin, il peut réaliser l'injection du fluide de comblement F1. Pour ce faire, le clapet 70 est placé en position fermée et le premier piston 110 est poussé vers l'avant. Le fluide de comblement F1 s'écoule alors entre le premier port d'entrée 601 et le port de sortie 602, pour être injecté depuis l'aiguille 200.

Figure 5d. En pratique, le fluide de comblement F1 est injecté dans plusieurs sites d'injection de l'individu. Entre deux injections, l'utilisateur purge l'aiguille 200 du fluide de comblement F1 de manière à ce qu'il puisse effectuer à nouveau l'aspiration in-vivo. Pour ce faire, le clapet 70 est placé en position ouverte, et le second piston 310 est poussé vers l'avant. L'aiguille 200 étant ouverte à son extrémité distale, la résistance du fluide de comblement F1 présent dans l'aiguille 200 est moins élevée que celle du fluide de comblement F1 présent dans le conduit C, dans la portion située entre le port de sortie 602 et le premier port d'entrée 601. Aussi, en poussant vers l'avant le second piston 310, la solution saline F2 coulera dans l'aiguille 200 et la purgera du fluide de comblement F1. Seul un très faible volume de fluide de comblement F1 est ainsi perdu. Après cette purge, on retrouve alors une configuration similaire à celle de la figure 5b, permettant de réaliser de manière sûre l'aspiration in-vivo.

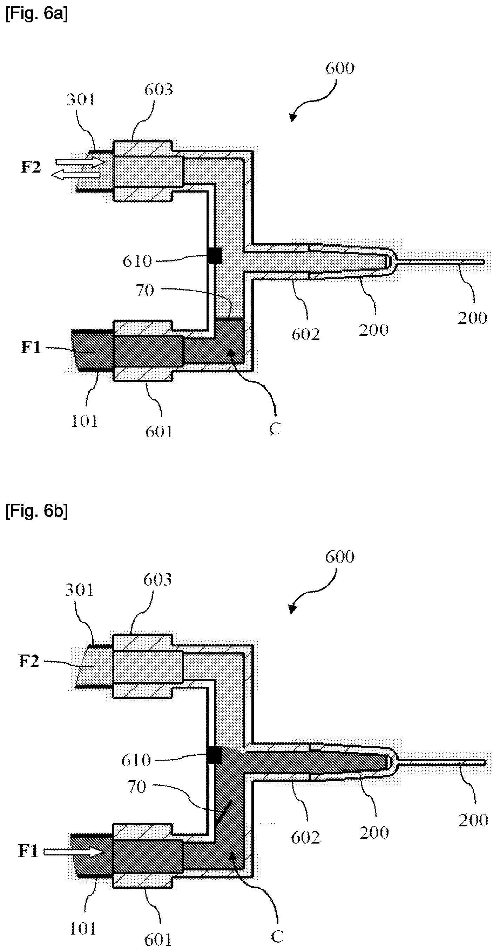

### Deuxième mode de réalisation : figures 6a et 6b

Le clapet 70 est disposé au niveau du premier port d'entrée 601, dans la branche du conduit C associée audit premier port. L'autre branche du conduit C associée au second port d'entrée 603 est libre. Ce clapet 70 ainsi que son mode d'actionnement, sont similaires à ceux décrits précédemment.

Figure 6a. Pour purger l'air dans l'aiguille 200, le clapet 70 est maintenu en position fermée, et le second piston 310 est poussé vers l'avant de manière à ce que la solution saline F2 s'écoule dans l'aiguille 200 et la purge d'air.

L'aspiration in-vivo est réalisée de la même façon que celle décrite en référence à la figure 5b. Le clapet 70 reste fermé et le second piston 310 est tiré vers l'arrière de manière à provoquer une aspiration d'un échantillon de fluide corporel au travers de l'aiguille 200. Cette aspiration in-vivo peut également être réalisée en maintenant ouvert le clapet 70 et en tirant vers l'arrière le premier piston 110 de manière à créer une dépression dans le conduit C, au niveau du premier port d'entrée 601.

Figure 6b. L'injection du fluide de comblement F1 dans le site d'injection est réalisée en plaçant le clapet 70 en position ouverte et en poussant vers l'avant le premier piston 110.

Pour purger l'aiguille 200 du fluide de comblement F1 entre deux injections, le clapet 70 est placé en position fermée, et le second piston 310 est poussé vers l'avant. Comme expliqué en référence à la figure 5d, la solution saline F2 coule dans l'aiguille 200 et la purge du fluide de comblement F1.

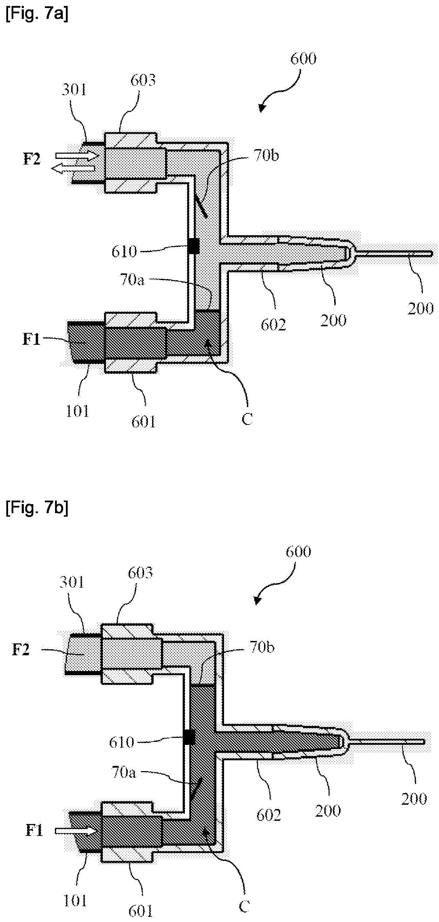

### Troisième mode de réalisation : figures 7a et 7b

L'adaptateur 600 comprend deux clapets 70a, 70b. Le premier clapet 70a est disposé au niveau du premier port d'entrée 601, dans la branche du conduit C associée audit premier port. Le second clapet 70b est disposé au niveau du second port d'entrée 603, dans la branche du conduit C associée audit second port. Chacun de ces clapets 70a, 70b ainsi que leur mode d'actionnement, sont similaires à ceux décrits précédemment. Ils peuvent également se présenter sous la forme de clapet anti-retour.

Figure 7a. Pour purger l'air dans l'aiguille 200, le premier clapet 70a est fermé et le second clapet 70a est ouvert. En poussant vers l'avant le second piston 310, la solution saline F2 s'écoule dans l'aiguille 200 et la purge d'air.

L'aspiration in-vivo est réalisée de la même façon que celle décrite en référence à la figure 5b. Le premier clapet 70a reste fermé et le second clapet 70b reste ouvert. Le second piston 310 est tiré vers l'arrière de manière à provoquer une aspiration d'un échantillon de fluide corporel au travers de l'aiguille 200. Cette aspiration in-vivo peut également être réalisée en maintenant ouvert le premier clapet 70a, en fermant le second clapet 70b et en tirant vers l'arrière le premier piston 110 de manière à créer une dépression dans le conduit C, au niveau du premier port d'entrée 601.

Figure 7b. L'injection du fluide de comblement F1 dans le site d'injection est réalisée en ouvrant le premier clapet 70a, en fermant le second clapet 70b et en poussant vers l'avant le premier piston 110.

Pour purger l'aiguille 200 du fluide de comblement F1 entre deux injections, le premier clapet 70a est fermé, le second clapet 70b est ouvert, et le second piston 310 est poussé vers l'avant. Comme expliqué en référence à la figure 5d, la solution saline F2 coule dans l'aiguille 200 et la purge du fluide de comblement F1.

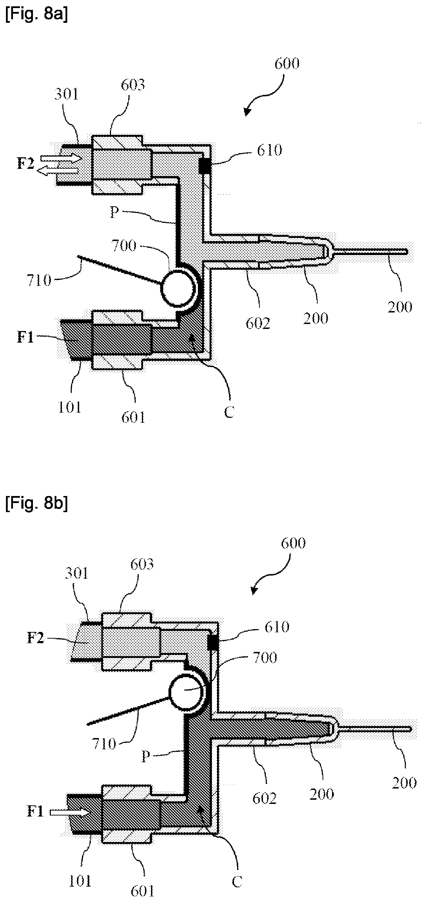

### Quatrième mode de réalisation : figures 8a et 8b

Le conduit C comporte au moins une paroi P déformable élastiquement. Cette paroi P est réalisée dans un matériau plastique déformable élastiquement tel qu'un thermoplastique souple à base de PVC, de FEP (Fluorinated Ethylene Propylene), de PTFE (polytétrafluoroéthylène) de silicone, ou un caoutchouc. Pour simplifier la conception, l'ensemble du conduit C est réalisé dans un tel matériau déformable élastiquement.

A la manière d'une pompe péristaltique, un organe presseur 700 permet d'aplatir la paroi déformable P de manière à obturer localement le conduit C. Celui-ci s'aplatie localement puis reprend, du fait de son élasticité, sa forme initiale. Selon un mode de réalisation, l'organe presseur 700 se présente sous la forme d'un galet ou d'un rouleau.

L'organe presseur 700 aplati la paroi déformable P dans deux zones distinctes : une première zone (figure 8a) pour empêcher la communication fluidique entre le premier port d'entrée 601 et le port de sortie 602 ; et une seconde zone (figure 8b) pour empêcher la communication fluidique entre le second port d'entrée 603 et le port de sortie 602. La première zone est située du côté du premier port d'entrée 601, dans la branche du conduit C associée audit premier port. A l'inverse, la seconde zone est située du côté du second port d'entrée 601, dans la branche du conduit C associée audit second port.

L'organe presseur 700 est relié à un actionneur 710 adapté pour le déplacer entre les deux zones d'aplatissement. Selon un mode de réalisation, l'actionneur 710 est un actionneur mécanique, par exemple relié à un bouton poussoir ou une molette manœuvré(e) manuellement, adapté pour déplacer l'organe presseur 700 entre les différentes zones d'aplatissement. Selon une variante de réalisation, l'actionneur mécanique 710 (et/ou l'organe presseur 700) coopère avec les dispositifs de verrouillage du type Luer-Lock permettant de connecter les extrémités proximale 105, 305 aux ports d'entrée 601, 603. En faisant pivoter dans un sens le dispositif de verrouillage associé au premier corps de seringue 101, par exemple de ¼ de tour ou ½ tour dans le sens horaire, l'organe presseur 700 agit dans la première zone. Et en faisant pivoter ce dispositif dans l'autre sens, par exemple de ¼ de tour ou ½ tour dans le sens anti-horaire, l'organe presseur 700 agit dans la seconde zone. On peut également prévoir que c'est en faisant pivoter le dispositif de verrouillage associé au second corps de seringue 301 qu'on déplace l'organe presseur 700 dans la seconde zone.

Selon un autre mode de réalisation, l'organe presseur 700 est déplacé au moyen d'un actionneur électromécanique 710. Ce dernier peut se présenter sous la forme d'un moteur électrique ou d'un électro-aimant, dont l'alimentation en courant est pilotée par l'unité de gestion électronique 440. Dans ce cas, le déplacement de l'organe presseur 700 entre les deux zones d'aplatissement peut être commandé par l'activation d'un bouton de commande dédié 450 installé sur le châssis 400 (figures 2 et 3). L'actionneur 710 peut également se présenter sous la forme d'un actionneur hydraulique ou pneumatique et se présenter par exemple sous la forme d'un vérin dont la tige est reliée à l'organe presseur 700.

Figure 8a. Pour purger l'air dans l'aiguille 200, l'organe presseur 700 est placé de manière à agir dans la première zone d'aplatissement. En poussant vers l'avant le second piston 310, la solution saline F2 s'écoule dans l'aiguille 200 et la purge d'air.

L'aspiration in-vivo est réalisée de la même façon que celle décrite en référence à la figure 5b. L'organe presseur 700 reste actif dans la première zone d'aplatissement. Le second piston 310 est tiré vers l'arrière de manière à provoquer une aspiration d'un échantillon de fluide corporel au travers de l'aiguille 200. Cette aspiration in-vivo peut également être réalisée en déplaçant l'organe presseur 700 de manière à ce qu'il agisse dans la seconde zone d'aplatissement et en tirant vers l'arrière le premier piston 110 de manière à créer une dépression dans le conduit C, au niveau du premier port d'entrée 601.

Figure 8b. L'injection du fluide de comblement F1 dans le site d'injection est réalisée en manœuvrant l'organe presseur 700 de manière à ce qu'il agisse dans la seconde zone d'aplatissement et en poussant vers l'avant le premier piston 110.

Pour purger l'aiguille 200 du fluide de comblement F1 entre deux injections, l'organe presseur 700 est à nouveau placé de manière à agir dans la première zone d'aplatissement (figure 8a), et le second piston 310 est poussé vers l'avant. Comme expliqué en référence à la figure 5d, la solution saline F2 coule dans l'aiguille 200 et la purge du fluide de comblement F1.

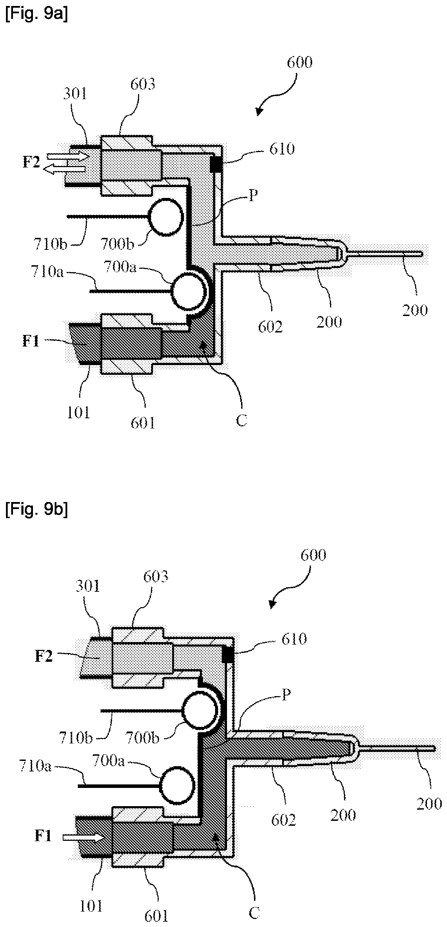

### Cinquième mode de réalisation : figures 9a et 9b

Ce mode de réalisation est similaire au quatrième mode décrit précédemment. Deux organes presseurs 700a, 700b sont toutefois utilisés. Un premier organe presseur 700a est adapté pour aplatir la paroi déformable P du conduit C dans la première zone (figure 9a) de manière à empêcher la communication fluidique entre le premier port d'entrée 601 et le port de sortie 602. Le second organe presseur 700b est adapté pour aplatir la paroi déformable P du conduit dans la seconde zone (figure 9b) de manière à empêcher la communication fluidique entre le second port d'entrée 603 et le port de sortie 602.

Chacun de ces organes presseurs 700a, 700b ainsi que leur mode d'actionnement, sont similaires à ceux décrits précédemment. Les actionneurs 700a, 700b peuvent toutefois être configurés pour simplement effectuer un mouvement de translation avant/arrière. En position avancée, l'organe presseur 700a, 700b aplatie la paroi déformable P, et en position reculée, il permet à ladite paroi de reprendre sa forme initiale. Dans la suite de la description, et par souci de concision, un organe presseur est dit « actif » ou « activé » lorsqu'il aplati la paroi déformable P, et « inactif » ou « inactivé » dans le cas contraire.

Figure 9a. Pour purger l'air dans l'aiguille 200, le premier organe presseur 700a est actif dans la première zone d'aplatissement, le second organe presseur 700b étant inactif dans la seconde zone d'aplatissement. En poussant vers l'avant le second piston 310, la solution saline F2 s'écoule dans l'aiguille 200 et la purge d'air.

L'aspiration in-vivo est réalisée de la même façon que celle décrite en référence à la figure 5b. Le premier organe presseur 700a reste actif et le second organe presseur 700b inactif. Le second piston 310 est tiré vers l'arrière de manière à provoquer une aspiration d'un échantillon de fluide corporel au travers de l'aiguille 200. Cette aspiration in-vivo peut également être réalisée en activant le second organe presseur 700b, en inactivant le premier organe presseur 700a et en tirant vers l'arrière le premier piston 110 de manière à créer une dépression dans le conduit C, au niveau du premier port d'entrée 601.

Figure 9b. L'injection du fluide de comblement F1 dans le site d'injection est réalisée en inactivant le premier organe presseur 700a, en activant le second organe presseur 700b et en poussant vers l'avant le premier piston 110.

Pour purger l'aiguille 200 du fluide de comblement F1 entre deux injections, on active le premier organe presseur 700a, on désactive le second organe presseur 700b (figure 9a), et on pousse vers l'avant le second piston 310. Comme expliqué en référence à la figure 5d, la solution saline F2 coule dans l'aiguille 200 et la purge du fluide de comblement F1.

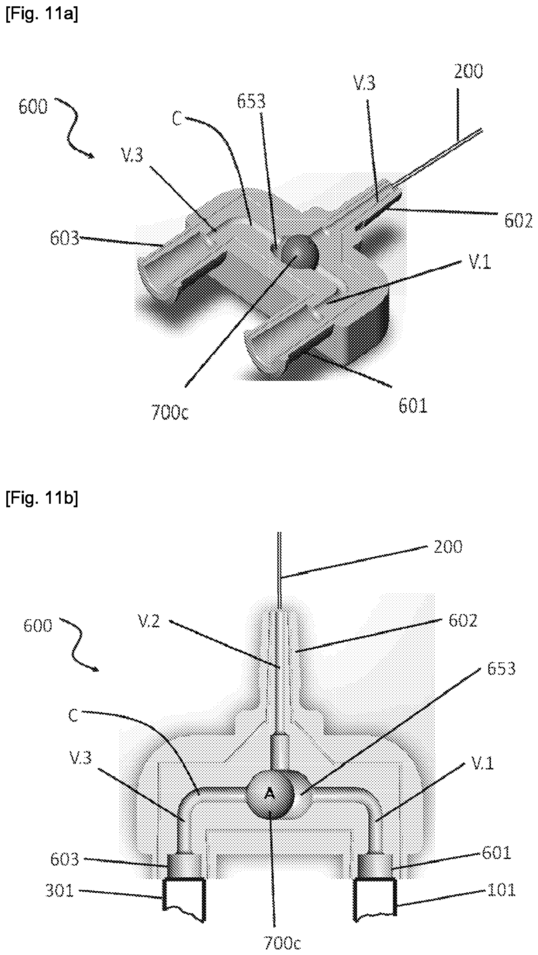

### Sixième mode de réalisation : figures 11a à 11c

L'adaptateur 600 comprend une cavité 650 aménagée à l'intersection entre les trois voies (ou branches) de communication fluide V.1, V.2, V.3 du conduit C.

La cavité 650 présente un premier orifice relié à la voie V.1 du conduit C associée au premier port d'entrée 601, un second orifice relié à la voie V.3 du conduit C associée au premier port d'entrée 601, et un troisième orifice relié à la voie V.2 du conduit C associée au port de sortie 602.

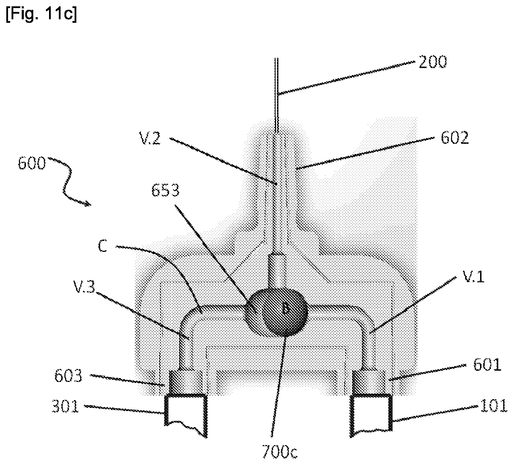

La cavité 650 loge un obturateur 700c. Celui-ci se présente sous la forme d'un élément mobile monté coulissant à l'intérieur de la cavité 650 entre une première position dans laquelle il obture la communication fluidique entre le second port d'entrée 603 et le port de sortie 602 lors du refoulement du premier produit injectable F1 hors du premier corps de seringue 101 en vue d'injecter le premier produit injectable F1 dans le site d'injection corporel (figure 11b), et une seconde position dans laquelle il obture la communication fluidique entre le premier port d'entrée 601 et le port de sortie 602 lors de l'aspiration de l'échantillon de fluide corporel dans le second corps de seringue 301 (figure 11c).

La section transversale de la cavité 650 correspond à la section transversale de l'élément mobile de telle sorte que celui-ci peut coulisser dans la cavité 650.

Selon un mode de réalisation, la cavité 650 a une forme générale tubulaire de section sensiblement circulaire ou oblongue et l'élément mobile représentant l'obturateur 700c a une forme de section transversale correspondant sensiblement à la section transversale de la cavité 650 (par exemple en forme de sphère ou de bille pour une cavité 650 de section circulaire).

L'élément mobile représentant l'obturateur 700c peut être réalisé dans un matériau plastique, en acier inoxydable, en verre, en céramique, ou dans tout autre matériau convenant à l'homme du métier.

Figure 11 b. Pour injecter le premier produit injectable F1 dans le site d'injection, le premier piston 101 est poussé vers l'avant. Le produit injectable F1 sortant du premier corps de seringue 101 entre dans la cavité 650 via le premier orifice relié à la voie V.1 du conduit C et pousse l'obturateur 700c (ou élément mobile), dans la cavité 650, en direction du second orifice qui est relié à la voie V.3 du conduit C afin d'obturer la communication fluidique entre le second port d'entrée 603 et le port de sortie 602. La communication fluidique entre le premier port d'entrée 601 et le port de sortie 602 est alors autorisée pour permettre d'injecter le premier produit injectable F1 dans le site d'injection.

Figure 11 c. Pour réaliser l'aspiration *in-vivo* de l'échantillon de fluide corporel du site d'injection, le second piston 310 est tiré vers l'arrière de manière à créer une dépression dans la voie V.1 du conduit C moyennant quoi l'obturateur 700c (ou élément mobile) est aspiré, à l'intérieur de la cavité 650, en direction du premier orifice relié à la voie V.1 afin d'obturer la communication fluidique entre le premier port d'entrée 601 et le port de sortie 602. La communication fluidique entre le second port d'entrée 603 et le port de sortie 602 est alors autorisée pour permettre l'aspiration de l'échantillon corporel du site d'injection, dans ledit second corps de seringue 301 à travers la aiguille creuse 200 et l'adaptateur 600. Ensuite, le second le second piston 310 est tiré vers l'arrière de manière à créer une dépression à l'extrémité distale de la aiguille creuse (200) à l'effet de laquelle l'échantillon de fluide corporel est aspiré dans le second corps de seringue 301 à travers la aiguille creuse 200 et l'adaptateur 600 (voies V2 + V3). Ainsi, l'échantillon de fluide corporel peut être aspiré sans risque de se mélanger avec le premier fluide injectable F1 (ex produit de comblement).

En résumé, en manipulant l'obturateur 70, 70a, 70b, 700, 700a, 700b, 700c, l'utilisateur peut aisément isoler l'un ou l'autre des corps de seringue, pour contrôler le produit à faire passer au travers de l'aiguille unique. Notamment, l'aiguille peut être purgée avec un produit neutre, particulièrement bon marché, contenu dans le second corps de seringue. Le produit actif ne sert que pour le traitement de l'individu, de sorte que son utilisation est optimisée en termes de quantité.

L'agencement des différents éléments et/ou moyens et/ou étapes de l'invention, dans les modes de réalisation décrits ci-dessus, ne doit pas être compris comme exigeant un tel agencement dans toutes les implémentations. D'autres variantes peuvent être prévues et notamment :
- La seringue peut comporter plus de deux corps de seringue, par exemple trois ou quatre, contenant chacun un produit distinct. L'adaptateur 600 présente alors d'autres ports d'entrée permettant leur connexion.
- Les ports d'entrée 601, 603 peuvent être fixés de manière indémontable sur les corps de seringue 101, 301, par exemple par moulage, collage ou soudage.
- L'aiguille 200 peut être installée de manière indémontable sur l'adaptateur 600.
- Le premier produit F1 peut se présenter sous la forme de poudre (par exemple destiné à être mélangée avec le second produit F2 dans l'adaptateur) ou sous la forme de gaz (par exemple un gaz à usage médical tel que l'ozone). Il en est de même pour le second produit F2.
- Le second produit F2 peut consister en un produit de comblement dermique injectable (liquide ou gel), une formulation médicale injectable, etc.
l'injection de trois fluides ou de quatre fluides par exemple.
- Les moyens d'entraînement 410, 430 peuvent se présenter sous la forme d'actionneurs pneumatiques ou hydrauliques (par exemple de type vérins), ou d'actionneur électromagnétique (par exemple du type électroaimants).
- Dans une application où le produit actif F1 doit être injecté dans la circulation sanguine (injection intraveineuse), l'étape d'aspiration in-vivo peut être exécutée pour s'assurer que l'échantillon de fluide corporel aspiré est bien du sang.
- L'adaptateur 600 peut comporter un clapet 70 dans une banche du conduit C et un organe presseur 700 dans l'autre branche.

En outre, une ou plusieurs caractéristiques exposées seulement dans un mode de réalisation peuvent être combinées avec une ou plusieurs autres caractéristiques exposées seulement dans un autre mode de réalisation. De même, une ou plusieurs caractéristiques exposées seulement dans un mode de réalisation peuvent être généralisées aux autres modes de réalisation.

## Revendications

1. Seringue d'injection (S) d'un premier produit injectable (F1) dans un site d'injection corporel d'un individu, comportant :
- un premier corps de seringue (101) contenant le premier produit injectable (F1), un premier piston (110) étant monté déplaçable dans ledit premier corps de seringue (101) ;
- une aiguille creuse (200) adaptée pour s'introduire dans ledit site d'injection corporel ;
- un adaptateur (600) connecté audit premier corps de seringue (101) et à ladite aiguille (200),
ladite seringue d'injection (S) comprenant en outre un dispositif de vérification permettant de s'assurer que, lors de l'injection du premier produit injectable (F1), ladite aiguille creuse (200) ne se trouve pas introduite dans un vaisseau sanguin, ledit dispositif de vérification comportant :
- un second corps de seringue (301) contenant un second produit (F2) et étant connecté audit adaptateur (600) ;
- un second piston (310) étant monté déplaçable dans ledit second corps de seringue (301) de manière à provoquer une dépression à l'extrémité distale de ladite aiguille creuse (200) moyennant quoi un échantillon de fluide corporel est aspiré dans ledit second corps de seringue (301) à travers ladite aiguille creuse (200) et ledit adaptateur (600) et se mélange avec le second produit (F2) contenu dans ledit second corps de seringue (301) en donnant une coloration rouge si ladite aiguille creuse (200) se trouve introduite dans un vaisseau sanguin et que le fluide corporel aspiré est du sang ;
l'adaptateur (600) comprenant :
- un premier port d'entrée (601) en communication fluidique avec le premier corps de seringue (101),
- un second port d'entrée (603) en communication fluidique avec le second corps de seringue (301),
- un port de sortie (602) en communication fluidique avec l'aiguille (200),
- un conduit (C) adapté pour mettre en communication fluidique le premier port d'entrée (601), le deuxième port d'entrée (603) et le port de sortie (602), le conduit (C) comprenant une cavité (650) aménagée à l'intersection entre les trois voies de communication fluide (V.1, V.2, V.3) du conduit (C) et un obturateur (700c) se présentant sous la forme d'un élément mobile monté coulissant à l'intérieur de ladite cavité (650) entre une première position à laquelle il obture la communication fluidique entre le second port d'entrée (603) et le port de sortie (602) lors du refoulement du premier produit injectable (F2) hors du premier corps de seringue (101) en vue d'injecter le premier produit injectable (F2) dans le site d'injection corporel, et une seconde position à laquelle il obture la communication fluidique entre le premier port d'entrée (601) et le port de sortie (602) lors de l'aspiration de l'échantillon de fluide corporel dans le second corps de seringue (301), la cavité (650) ayant une forme générale tubulaire ou oblongue de section sensiblement circulaire et l'élément mobile représentant l'obturateur (700c) ayant une section transversale correspondant sensiblement à la section transversale de la cavité 650,
de sorte que, lorsque le premier piston (110) est poussé vers l'avant, le premier produit injectable (F1 ) sort du premier corps de seringue (101), entre dans la cavité (650) via un premier orifice relié à une première voie de communication fluidique (V.1) du conduit (C) et pousse l'obturateur (700c) dans la cavité (650), en direction d'un second orifice qui est relié à une deuxième voie de communication fluidique (V.3) du conduit (C) jusqu'à la première position pour permettre d'injecter le premier produit injectable (F1) dans le site d'injection,
**caractérisée en ce que** lorsque le second piston (310) est tiré vers l'arrière de manière à créer une dépression dans la première voie de communication fluidique (V.1) du conduit (C) l'obturateur (700c) est aspiré, à l'intérieur de la cavité (650), en direction du premier orifice relié à la première voie de communication fluidique (V.1) jusqu'à la deuxième position pour permettre l'aspiration de l'échantillon corporel du site d'injection dans ledit second corps de seringue (301) à travers la aiguille creuse (200) et l'adaptateur (600) de sorte que lorsque le second le second piston (310) est ensuite tiré vers l'arrière, de manière à créer une dépression à l'extrémité distale de la aiguille creuse (200), l'échantillon de fluide corporel est aspiré dans le second corps de seringue (301) à travers la aiguille creuse (200) et l'adaptateur (600).

2. Seringue selon la revendication précédente, dans laquelle le premier piston (110) et le second piston (310) sont mus par des moyens d'entraînement motorisés (410, 430).

3. Seringue selon l'une des revendications précédentes, dans laquelle un détecteur de sang (610) est installé dans l'adaptateur (600) et/ou dans le premier corps de seringue (101) et/ou dans le second corps de seringue (301).

4. Seringue selon l'une des revendications précédentes, dans laquelle le second corps de seringue (301) contient le second produit (F2), et dans laquelle la communication fluidique entre le second port d'entrée (603) et le port de sortie (602) permet une aspiration d'un échantillon de fluide corporel au niveau du site d'injection, au travers de l'aiguille (200), lorsque :
- à pression et température ambiantes, le second produit (F2) a une viscosité dynamique comprise entre 10⁻³ N.m⁻².s et 10⁻² N.m⁻².s,
- et le second piston (310) est tiré dans le second corps de seringue (301).

5. Seringue selon l'une des revendications précédentes, dans laquelle le premier corps de seringue (101) contient le premier produit injectable (F1) et le second corps de seringue (301) contient le second produit (F2), et dans laquelle à pression et température ambiantes, le second produit (F2) a une viscosité dynamique plus faible que celle du premier produit (F1).

6. Seringue selon l'une des revendications précédentes, dans laquelle le premier corps de seringue (101) contient le premier produit injectable (F1) et le second corps de seringue (301) contient le second produit (F2), et dans laquelle le premier produit (F1) est un produit de comblement dermique se présentant sous la forme d'une solution injectable ou une formulation médicale se présentant sous la forme d'une solution injectable.

7. Seringue selon la revendication 4, dans laquelle le produit de comblement dermique (F1) comprend au moins une substance sélectionnée dans le groupe constitué de: acide hyaluronique, collagène, acide poly-lactique, gel de polyacrylamide, hydroxyapatite de calcium, microsphères de calcium hydroxyl appétit en suspension dans un support de gel aqueux, phosphate tricalcique, microsphères de polyméthacrylate de méthyle, billes de dextran, billes de dextran suspendues dans du gel hylan d'origine non animale, peptides d'élastine solubilisés avec du collagène bovin, silicone, acide poly-L-lactique, polytétrafluoroéthylène (PTFE), polytétrafluoroéthylène expansé (e-PTFE) et au moins l'une de leurs combinaisons.

8. Seringue selon l'une des revendications 4 à 5, dans laquelle le produit de comblement dermique (F1) comprend un ou plusieurs agents biologiquement actifs tels que les toxines botuliques, les anesthésiants locaux, les corticoïdes locaux, ou les antibiotiques, les acides aminés, les antioxydants.

9. Seringue selon la revendication 4, dans laquelle la formulation médicale injectable (F1) comprend un ou plusieurs agents biologiquement actifs sélectionnés dans le groupe constitué de : toxines botuliques, anesthésiants locaux, corticoïdes locaux, antibiotiques, acides aminés, antioxydants, produits opacifiants, et au moins l'une de leurs combinaisons.

10. Seringue selon l'une des revendications précédentes, dans laquelle le second corps de seringue (301) contient le second produit (F2), et dans laquelle le second produit (F2) est une solution liquide saline physiologique.

## Patentansprüche

1. Spritze (S) zur Injektion eines ersten injizierbaren Produkts (F1) in eine Körper-Injektionsstelle einer Person, umfassend:
- einen ersten Spritzenkörper (101), der das erste injizierbare Produkt (F1) enthält, wobei ein erster Kolben (110) beweglich in dem ersten Spritzenkörper (101) montiert ist;
- eine Hohlnadel (200), die zum Einführen in die Körper-Injektionsstelle geeignet ist;
- einen Adapter (600), der mit dem ersten Spritzenkörper (101) und der Nadel (200) verbunden ist,
wobei die Injektionsspritze (S) ferner eine Prüfvorrichtung umfasst, mit der sichergestellt werden kann, dass sich die Hohlnadel (200) bei der Injektion des ersten injizierbaren Produkts (F1) nicht in einem Blutgefäß befindet,
wobei die Prüfvorrichtung Folgendes umfasst:
- einen zweiten Spritzenkörper (301), der ein zweites Produkt (F2) enthält und mit diesem Adapter (600) verbunden ist;
- einen zweiten Kolben (310), der beweglich in dem zweiten Spritzenkörper (301) montiert ist, so dass er einen Unterdruck am distalen Ende der Hohlnadel (200) verursacht, wodurch eine Körperflüssigkeitsprobe durch die Hohlnadel (200) und den Adapter (600) in den zweiten Spritzenkörper (301) angesaugt wird und sich mit dem zweiten Produkt (F2) vermischt, das in dem zweiten Spritzenkörper (301) enthalten ist, wodurch eine rote Färbung entsteht, wenn die Hohlnadel (200) in ein Blutgefäß eingeführt wurde und die abgesaugte Körperflüssigkeit Blut ist;
wobei der Adapter (600) umfasst:
- einen ersten Einlassanschluss (601) in Fluidverbindung mit dem ersten Spritzenkörper (101),
- einen zweiten Einlassanschluss (603) in Fluidverbindung mit dem zweiten Spritzenkörper (301),
- einen Auslassanschluss (602) in Fluidverbindung mit der Nadel (200),
- eine Leitung (C), die geeignet ist, den ersten Einlassanschluss (601), den zweiten Einlassanschluss (603) und den Auslassanschluss (602) in Fluidverbindung miteinander zu versetzen,
**dadurch gekennzeichnet, dass** die Leitung (C) einen Hohlraum (650) umfasst, der an der Kreuzung zwischen den drei Fluidverbindungskanälen (V.1, V.2, V.3) der Leitung (C) angeordnet ist, und einen Verschluss (700c) in Form eines montierten beweglichen Elements, der sich innerhalb des Hohlraums (650) zwischen einer ersten Position, in der er die Fluidverbindung zwischen dem zweiten Einlassanschluss (603) und dem Auslassanschluss (602) beim Fördern des ersten injizierbaren Produkts (F2) aus dem ersten Spritzenkörper (101) heraus verschließt, um das erste injizierbare Produkt (F2) in die Körper-Injektionsstelle zu injizieren, und einer zweiten Position, in der er die Fluidverbindung zwischen dem ersten Einlassanschluss (601) und dem Auslassanschluss (602) beim Ansaugen der Körperflüssigkeitsprobe in den zweiten Spritzenkörper (301) verschließt, verschiebt, wobei der Hohlraum (650) allgemein röhrenartig oder länglich geformt ist sowie im Wesentlichen einen kreisförmigen Querschnitt aufweist und das den Verschluss (700c) darstellende, bewegliche Element einen Querschnitt aufweist, der im Wesentlichen dem Querschnitt des Hohlraums 650 entspricht,
sodass beim Drücken des ersten Kolbens (110) nach vorne das erste injizierbare Produkt (F1) aus dem ersten Spritzenkörper (101) austritt, über eine erste mit einem ersten Fluidverbindungskanal (V.1) der Leitung (C) verbundene Öffnung in den Hohlraum (650) eintritt und den Verschluss (700c) im Hohlraum (650) bis zur ersten Position in Richtung einer zweiten Öffnung drückt, die mit einem zweiten Fluidverbindungskanal (V.3) der Leitung (C) verbunden ist, um das erste injizierbare Produkt (F1) in die Injektionsstelle injizieren zu können,
und sodass beim Zurückziehen des zweiten Kolbens (310) zum Erzeugen eines Unterdrucks im ersten Fluidverbindungskanal (V.1) der Leitung (C) der Verschluss (700c) innerhalb des Hohlraums (650) in Richtung der ersten Öffnung, die mit dem ersten Fluidverbindungskanal (V.1) verbunden ist, bis zur zweiten Position angesaugt wird, um das Ansaugen der Körperprobe der Injektionsstelle in den zweiten Spritzenkörper (301) durch die Hohlnadel (200) und den Adapter (600) zu ermöglichen, sodass beim anschließenden Zurückziehen des zweiten Kolbens (310) zum Erzeugen eines Unterdrucks am distalen Ende der Hohlnadel (200) die Körperflüssigkeitsprobe durch die Hohlnadel (200) und den Adapter (600) in den zweiten Spritzenkörper (301) gesaugt wird.

2. Spritze nach dem vorhergehenden Anspruch, wobei der erste Kolben (110) und der zweite Kolben (310) durch motorische Antriebsmittel (410, 430) bewegt werden.

3. Spritze nach einem der vorhergehenden Ansprüche, wobei ein Blutdetektor (610) im Adapter (600) und/oder im ersten Spritzenkörper (101) und/oder im zweiten Spritzenkörper (301) installiert ist.

4. Spritze nach einem der vorhergehenden Ansprüche, wobei der zweite Spritzenkörper (301) das zweite Produkt (F2) enthält, und wobei die Fluidverbindung zwischen dem zweiten Einlassanschluss (603) und dem Auslassanschluss (602) die Ansaugung einer Körperflüssigkeitsprobe an der Injektionsstelle durch die Nadel (200) ermöglicht, wenn:
- das zweite Produkt (F2) bei Raumdruck und -temperatur eine dynamische Viskosität zwischen 10⁻³ N.m⁻².s und 10⁻² N.m⁻².s hat,
- und der zweite Kolben (310) in den zweiten Spritzenkörper (301) gezogen wird.

5. Spritze nach einem der vorhergehenden Ansprüche, wobei der erste Spritzenkörper (101) das erste injizierbare Produkt (F1) und der zweite Spritzenkörper (301) das zweite Produkt (F2) enthält, und wobei das zweite Produkt (F2) bei Raumdruck und -temperatur eine niedrigere dynamische Viskosität aufweist als das erste Produkt (F1).

6. Spritze nach einem der vorhergehenden Ansprüche, wobei der erste Spritzenkörper (101) das erste injizierbare Produkt (F1) und der zweite Spritzenkörper (301) das zweite Produkt (F2) enthält, und wobei das erste Produkt (F1) ein dermales Füllmittel in Form einer injizierbaren Lösung oder eine medizinische Rezeptur in Form einer injizierbaren Lösung ist.

7. Spritze nach Anspruch 4, wobei das dermale Füllmittel (F1) mindestens einen Stoff umfasst, der aus der Gruppe ausgewählt ist, bestehend aus: Hyaluronsäure, Kollagen, Polymilchsäure, Polyacrylamidgel, Kalziumhydroxyapatit, Kalziumhydroxylapatit-Mikrosphären in wässriger Gel-Suspension, Trikalziumphosphat, Polymethylmethacrylat-Mikrosphären, Dextran-Kugeln, Dextran-Kugeln in Hylangel nicht tierischen Ursprungs, mit Rinderkollagen gelöste Elastin-Peptide, Silikon, Poly-L-Milchsäure, Polytetrafluorethylen (PTFE), expandiertes Polytetrafluorethylen (e-PTFE) und mindestens eine ihrer Kombinationen.

8. Spritze nach einem der Ansprüche 4 bis 5, wobei das dermale Füllmittel (F1) einen oder mehrere biologisch aktive Wirkstoffe wie Botulinumtoxine, Lokalanästhetika, Lokalkortikoide oder Antibiotika, Aminosäuren, Antioxidantien umfasst.

9. Spritze nach Anspruch 4, wobei die injizierbare medizinische Rezeptur (F1) einen oder mehrere biologisch aktive Wirkstoffe umfasst, die aus der Gruppe ausgewählt sind, bestehend aus: Botulinumtoxine, Lokalanästhetika, Lokalkortikoide, Antibiotika, Aminosäuren, Antioxidantien, Trübungsmittel, und mindestens eine ihrer Kombinationen.

10. Spritze nach einem der vorhergehenden Ansprüche, wobei der zweite Spritzenkörper (301) das zweite Produkt (F2) enthält, und wobei das zweite Produkt (F2) eine physiologische flüssige Kochsalzlösung ist.

## Claims

1. Injection syringe (S) for injecting a first injectable product (F1) into a body injection site of an individual, comprising:
- a first syringe barrel (101) containing the first injectable product (F1), a first plunger (110) being displaceably mounted in said first syringe barrel (101);
- a hollow needle (200) suited to being inserted into said body injection site;
- an adapter (600) connected to said first syringe barrel (101) and said needle (200),
said injection syringe (S) further comprising a verification device making it possible to ensure that, when the first injectable product (F1) is injected, said hollow needle (200) is not introduced into a blood vessel, said verification device comprising:
- a second syringe barrel (301) containing a second product (F2) and being connected to said adapter (600);
- a second plunger (310) being displaceably mounted in said second syringe barrel (301) so as to cause a depression at the distal end of said hollow needle (200) whereby a sample of body fluid is aspirated into said second syringe barrel (301) through said hollow needle (200) and said adapter (600) and is mixed with the second product (F2) contained in said second syringe barrel (301) giving a red color if said hollow needle (200) is inserted into a blood vessel and the aspirated fluid is blood,
the adapter (600) comprising:
- a first inlet port (601) in fluidic communication with the first syringe barrel (101),
- a second inlet port (603) in fluidic communication with the second syringe barrel (301),
- an outlet port (602) in fluidic communication with the needle (200),
- a conduit (C) suitable for placing the first inlet port (601), the second inlet port (603) and the outlet port (602) in fluidic communication,
the conduit (C) comprising a cavity (650) arranged at the intersection between the three fluid communication pathways (V.1, V.2, V.3) of the conduit (C) and a shutter (700c) being in the form of a movable element slidingly mounted within said cavity (650) between a first position at which it occludes the fluidic communication between the second inlet port (603) and the outlet port (602) upon discharge of the first injectable product (F2) out of the first syringe barrel (101) for the purpose of injecting the first injectable product (F2) into the body injection site, and a second position at which it occludes the fluidic communication between the first inlet port (601) and the outlet port (602) when aspirating the fluid sample into the second syringe barrel (301), the cavity (650) has a general tubular shape or oblong of substantially circular cross-section and the movable element representing the shutter (700c) having a cross-sectional shape corresponding substantially to the cross-section of the cavity (650),
such that, when the first plunger (101) is pushed forward, the injectable product (F1) comes out of the first syringe barrel (101), enters the cavity (650) via the first orifice connected to the channel (V.1) of the conduit (C) and pushes the shutter (700c) into the cavity (650), in the direction of the second orifice that is connected to the channel (V.3) of the conduit (C) to the first position to enable the first injectable product (F1) to be injected into the injection site,
**characterized in that** when the second plunger (310) is pulled backwards to create a depression in the channel (V.1) of the conduit (C) whereby the shutter (700c) is aspirated, inside the cavity (650), in the direction of the first orifice connected to the channel (V.1) to the second position to enable the aspiration of the body sample from the injection site, into said second syringe barrel (301) through the hollow needle (200) and the adapter (600) such that when the second plunger (301) is then pulled back so as to create a depression at the distal end of the hollow needle (200), the sample of body fluid is aspirated in the second syringe barrel (301) toward the hollow needle (200) and the adapter (600).

2. Syringe according to one of the preceding claim, wherein the first plunger (110) and the second plunger (310) are driven by motorized drive means (410, 430).

3. Syringe according to one of the preceding claims, wherein a blood detector (610) is installed in the adapter (600) and/or in the first syringe barrel (101) and/or in the second syringe barrel (301).

4. Syringe according to one of the preceding claims, wherein the second syringe barrel (301) contains the second product (F2), and wherein the fluidic communication between the second inlet port (603) and the outlet port (602) allows aspiration of a body fluid sample at the injection site, through the needle (200), when:
- at ambient pressure and temperature, the second product (F2) has a dynamic viscosity of between 10⁻³ N.m⁻².s and 10⁻² N.m⁻².s,
- and the second plunger (310) is pulled into the second syringe barrel (301).

5. Syringe according to one of the preceding claims, wherein the first syringe barrel (101) contains the first injectable product (F1) and the second syringe barrel (301) contains the second product (F2), and wherein at ambient pressure and temperature, the second product (F2) has a lower viscosity than the first product (F1).

6. Syringe according to one of the preceding claims, wherein the first syringe barrel (101) contains the first injectable product (F1) and the second syringe barrel (301) contains the second product (F2), and wherein the first product (F1) is a dermal filler in the form of an injectable solution or a medical formulation in the form of an injectable solution.

7. Syringe according to claim 4, wherein the dermal filler (F1) comprises at least one substance selected from the group consisting of: hyaluronic acid, collagen, polylactic acid, polyacrylamide gel, calcium hydroxyapatite, calcium hydroxyapatite microspheres suspended in an aqueous gel carrier, tricalcium phosphate, polymethylmethacrylate microspheres, dextran beads, dextran beads suspended in hylan gel of non-animal origin, elastin peptides solubilized with bovine collagen, silicone, poly-L-lactic acid, polytetrafluoroethylene (PTFE), expanded polytetrafluoroethylene (e-PTFE), and at least one of the combinations thereof.

8. Syringe according to one of claims 4 to 5, wherein the dermal filler (F1) comprises one or more biologically active agents such as botulinum toxins, local anesthetics, local corticosteroids, or antibiotics, amino acids, antioxidants.

9. Syringe according to claim 12, wherein the injectable medical formulation (F1) comprises one or more biologically active agents selected from the group consisting of: botulinum toxins, local anesthetics, local corticosteroids, antibiotics, amino acids, antioxidants, opacifiers, and at least one of the combinations thereof.

10. Syringe according to one of the preceding claims, wherein the second syringe barrel (301) contains the second product (F2), and wherein the second product (F2) is a physiological liquid saline solution.
